# EUROPÄISCHE PATENTSCHRIFT

(11) **EP 1 292 266 B1**
(45) Veröffentlichungstag und Bekanntmachung des Hinweises auf die Patenterteilung: **16.11.2005**
(21) Anmeldenummer: 01964963.1
(22) Anmeldetag: 09.06.2001
(51) Int. Cl.: A61K 7/06, A61K 7/13

(54) **PYRIDOXIN ALS NEUE KUPPLERKOMPONENTE FÜR OXIDATIONSFÄRBEMITTEL**
PYRIDOXINE AS NOVEL COUPLING COMPONENT FOR OXIDATIVE HAIR DYES
PYRIDOXINE COMME NOUVEAU COUPLEUR POUR COLORANTS D'OXYDATION

(30) Priorität: 20.06.2000 DE 10030313; 26.04.2001 DE 10120307
(43) Veröffentlichungstag der Anmeldung: 19.03.2003
(73) Patentinhaber: Henkel Kommanditgesellschaft auf Aktien, 40589 Düsseldorf (DE)
(72) Erfinder: NAUMANN, Frank, 40219 Düsseldorf (DE); KLEEN, Astrid, 40699 Erkrath (DE); HÖFFKES, Horst, 40595 Düsseldorf (DE); MEINIGKE, Bernd, 51381 Leverkusen (DE)
(86) Internationale Anmeldenummer: PCT/EP2001/006556
(87) Internationale Veröffentlichungsnummer: WO 2001/097756

(56) Entgegenhaltungen:
- EP-A- 0 873 744
- EP-A- 1 101 823
- DE-A- 19 955 915
- CHEMICAL ABSTRACTS, vol. 107, no. 20, 16. November 1987 (1987-11-16) Columbus, Ohio, US; abstract no. 183330, H.KOJIMA, J.TAKENAKA: "Hair preparations containing reducing agents, sequestering agents, metallic salts, dyes and oxidizing agents" XP002183446 & JP 62 132813 A (NISHIRENJI TRADING) 16. Juni 1987 (1987-06-16)
- CHEMICAL ABSTRACTS, vol. 112, no. 8, 19. Februar 1990 (1990-02-19) Columbus, Ohio, US; abstract no. 62357, H.KOJIMA, J.TAKENAKA: "Simultaneous procedure for hair dyeing and wave setting" XP002184004 & JP 01 066109 A (SANSHIDO SEIYAKU) 13. März 1989 (1989-03-13)
- CHEMICAL ABSTRACTS, vol. 107, no. 20, 16. November 1987 (1987-11-16) Columbus, Ohio, US; abstract no. 183331, H.KOJIMA, J.TAKENAKA: "Hair preparations containing reducing agents, metallic salts, dyes and oxidizing agents" XP002184002 & JP 62 132814 A (SANSHIDO SEIYAKU) 16. Juni 1987 (1987-06-16)
- CHEMICAL ABSTRACTS, vol. 110, no. 14, 3. April 1989 (1989-04-03) Columbus, Ohio, US; abstract no. 121002, H.KOJIMA, J.TAKENAKA: "Hair preparations for wave setting and dyeing at the same time" XP002184003 & JP 63 014712 A (SANSHIDO SEIYAKU) 21. Januar 1988 (1988-01-21)
- PATENT ABSTRACTS OF JAPAN vol. 0185, no. 28, 6. Oktober 1994 (1994-10-06) & JP 06 183934 A (HOYU), 5. Juli 1994 (1994-07-05)
- ÜBERSETZUNG INS DEUTSCHE DER JAPANISCHEN OFFENLEGUNGSSCHRIFT 6-183934:
- "Römpp Chemie Lexikon, 9. Auflage, 1995, Bd.1, S. 686" * Seite 686 *

## Beschreibung

Die Erfindung betrifft Verfahren zum Färben von Keratinfasern, in welchen Pyridoxin oder eines des entsprechenden physiologish verträglichen Salze davon zur Anwendung kommt und Färbemittel mit solchen Verbindungen, sowie Kits zur Verwendung in dem erfindungsgemäßen Verfahren.

Unter Keratinfasern werden erfindungsgemäß Pelze, Wolle, Federn und insbesondere das menschliche Haar verstanden. Das menschliche Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintreten kann.

Für das Färben von Keratinfasern, insbesondere von Haaren, spielen die sogenannten Oxidationsfärbemittel wegen ihrer intensiven Farben und guten Echtheitseigenschaften, die bei relativ niedriger Färbetemperatur und in kurzen Färbezeiten erzielt werden, eine besondere Rolle. Solche Färbemittel enthalten in einem geeigneten, meist wäßrigen Träger eine Entwicklerkomponente, die unter dem Einfluß von Luftsauerstoff oder von Oxidationsmitteln durch oxidative Kupplung den Farbstoff ausbildet. Dieser Farbstoff kann durch Kupplung mit einer anderen Entwicklerkomponente oder mit sogenannten Kupplerkomponenten, die selbst keine Farbstoffe ausbilden können, intensiviert und in der Nuance modifiziert werden.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufzehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Reibung und den Einfluß chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Der Anwender von Haarfärbemitteln beabsichtigt unter anderem, eine natürlich wirkende Haarfarbe als Färbeergebnis zu erzielen. Das ist vor allem dann der Fall, wenn graues Haar unauffällig durch eine natürlich aussehende Färbung kaschiert werden soll. Zur Erzeugung natürlich wirkender Farbnuancen kommt den Haarfärbe- und Tönungsmitteln, die in den Farbnuancen im Rot- und Braunbereich färben, eine erhebliche Bedeutung zu. Oxidationsfärbemittel im Rot- und Braunbereich, wie sie z.B. mit der Kombination von 2,4,5,6-Tetraaminopyrimidin mit 2-Methylresorcin zugänglich sind, sind noch nicht optimal hinsichtlich der Gleichmäßigkeit des Farbaufzugs. Goldene Farbtöne waren unter Verwendung von herkömmlichen Kuppler- und Entwicklerkombinationen bisher nicht in befriedigender Weise erhältlich. Um eine große Variation an Farbtönen zu bekommen, können zur Nuancierung u.a. direktziehende Farbstoffe zur Anwendung kommen. Letztere sind meist weniger waschecht und daher nicht so gut zur Kombination mit Oxidationsfarbstoffen geeignet.
Eine natürlich erscheinende Haarfarbe geht ebenso aus einem Färbevorgang hervor, wenn in dem applizierten Haarfärbemittel Indol- oder Indolinderivate als Vorprodukte naturanaloger Farbstoffe verwendet werden. In der Druckschrift WO 9906016 A1 wird beschrieben, daß sich durch Verwendung einer Kombination von Derivaten des Indols oder Indolins mit üblichen Kupplerkomponenten teilweise bzw. total ergrautes Haar in die ursprüngliche natürliche Nuance zurücktönen läßt, so daß kein signifikanter Unterschied zu gegebenenfalls noch vorhandenem, natürlich pigmentiertem Haar sichtbar ist. Dabei werden blonde bis mittelbraune Ausfärbungen erzielt. Die in der Druckschrift WO 9906016 A1 veröffentlichten Farbstoffkombinationen erzielen dunkle bis schwarze Ausfärbungen ohne eine rote Nuance, die mit den als klassisch zu bezeichnenden Kuppler-Entwickler-Kombinationen nur schwer zugänglich waren.

Die Färbung von Keratinfasern unter Einsatz von naturanalogen Farbstoffen setzt einen Trend, natürlich erscheinende Färbungen auch mit natürlichen oder naturidentischen Farbstoffvorprodukten zu bewirken. Es ist daher wünschenswert, die Tönung des Haars durch Zusatz eines gleichfalls natürlichen oder naturidentischen Oxidationsfarbstoff-Vorprodukts vom Kuppler-Typ in der Farbgebung variieren zu können. Der Zugang zu einer breiteren Farbpalette sollte dadurch eröffhet und eine möglichst intensive, wasch- und lichtechte Färbung erzielt werden.

Neben der Optimierung der Farbgebung von Haarfärbemitteln ist die Verbesserung der Verträglichkeit der Mittel eine weitere Aufgabe. Oxidative Komponenten z.B. in Haarfärbemitteln wirken sich schädigend auf die Struktur des Haarkeratins aus. Das Haar erfährt einen Gewichtsverlust und eine meßbare Senkung der Denaturierungs-Temperatur des Keratins. Eine zunehmende Brüchigkeit und die erschwerte Kämmbarkeit des Haars sowie eine Verschlechterung von Sitz und Fülle der Frisur sind die Folge. Darüber hinaus weist ein strukturgeschädigtes Haar ein stumpfes und glanzloses Aussehen auf. Diesen Problemen sollte mit strukturverbessemden Zusatzstoffen im Rahmen des Färbeverfahrens, vorteilhafteiweise als Bestandteile des Färbemittels selbst, entgegengewirkt werden.

Aus diesen genannten Gründen besteht ein Bedarf an neuartigen Oxidationsfarbstoff-Vorprodukten und Pflegewirkstoffen, die eine Verbesserung der erwähnten Parameter ermöglichen. Überraschenderweise wurde gefunden, daß Pyridoxin oder eines des entsprechenden physiologish verträglichen Salze davon die entsprechenden Anforderungen in hervorragender Weise erfüllen.

Pyridoxin und weitere Verbindungen der Vitamin B6-Gruppe sind als Komponenten in Haartonika zur Verringerung des Nachfettens und zur Stimulierung des Haarwuchses erwähnt worden. In EP 0678293 A2 werden topische Zusammensetzungen mit einem Gehalt von Pyridoxintripropionat zur Behandlung des Haars und der Haut vorgeschlagen. In EP 001079 A1 sind kosmetische Zusammensetzungen mit antiseborrhoischer Wirkung beschrieben, die Pyridoxin-tripalmitat als Wirkstoff enthalten.

In der EP-873744 A2 werden Carbonylverbindungen, u.a. Pyridoxal, in Kombination mit Aminen, Hydroxyverbindungen, Peptiden, CH-aktiven Verbindungen und weiteren Komponenten als Farbstoffvorprodukte in bevorzugt nichtoxidativen Haarfärbemitteln offenbart.

Haarfärbemittel, die Derivate des Pyridoxins, Pyridoxals oder Pyridoxamins als wirksame Oxidationsfarbstoff-Vorprodukte und als strukturverbessernden Zusatzstoff enthalten, sind dem Fachmann bisher nicht bekannt.

Es wurde überraschend anhand von Vergleichsfärbungen gefunden, daß Pyridoxin Kupplereigenschaften aufweist. Insbesondere wurde gefunden, daß unter Einsatz dieser Verbindung als Kupplerkomponente in Haarfärbemitteln die Farbpalette um mittelgoldblonde Farben erweitert wird. Weiterhin wurde gefunden, daß die Verwendung der erfindungsgemäßen Verbindung während des Färbevorgangs eine Verbesserung der Struktur des Haarkeratins bewirkt.

Ein erster Gegenstand der Erfindung ist daher ein Verfahren zum Färben von Keratinfasern, insbesondere dem menschlichen Haar, bei dem
- gewünschtenfalls ein Vorbehandlungsmittel M1 auf die Faser aufgebracht wird, dann
- ein Färbemittel M2 ,enthaltend mindestens ein Indol- und/oder Indolinderivat und/oder ein Oxidationsfarbstoff-Vorprodukt vom Entwickler - Typ sowie ein Oxidationsmittel und/oder ein Enzgen, auf der Faser zur Anwendung kommt, das gegebenenfalls unmittelbar vor dem Auftragen auf die Faser aus:
   einer Komponente M2a, enthaltend mindestens ein Oxidationsfarbstoff-Vorprodukt vom Entwickler-Typ und/oder ein Indol- und/oder Indolinderivat und
   einer Komponente M2b, enthaltend ein Oxidationsmittel und/oder ein Enzym
gemischt wird, wobei gewünschtenfalls den einzelnen Mitteln M2a oder M2b vor der Mischung oder der Mischung M2 ein weiteres Mittel M3 zugegeben wird und dieses Färbemittel M2 nach einer Zeit von 5-30 Minuten von der Faser abgespült wird, dadurch gekennzeichnet, daß mindestens eines der Mittel M1, M2a, M2b oder M3 Pyridoxin oder eines des entsprechenden physiologish verträglichen Salze davon enthält. Pyridoxin ist käuflich zu erwerben.

Ein zweiter Gegenstand der Erfindung ist ein Mittel zur Verwendung in einem Verfahren zur Färbung von Keratinfasern, insbesondere dem menschlichen Haar, dadurch gekennzeichnet, daß es Pyridoxin oder eines der entsprechenden physiologisch verträglichen Salze enthält. Dieses Mittel fungiert als Vorbehandlungsmittel M1 in dem erfindungsgemäßen Verfahren. Bei Anwendung des Mittels M1 ist eine Einwirkungszeit von 1-30 Minuten bevorzugt.

Ein dritter Gegenstand der Erfindung ist ein Mittel zum Färben von Keratinfasern, insbesondere dem menschlichen Haar, enthaltend in einer ersten Ausführungsform mindestens ein Oxidationsmittel und / oder ein Einzgen sowie mindestens ein Oxidationsfarbstoff-Vorprodukt vom Entwickler-Typ, dadurch gekennzeichnet, daß es zusätzlich Pyridoxin oder eines der entsprechenden physiologisch verträglichen Salze enthält. Diese Mittel können gegebenenfalls mindestens ein Oxidationsfarbstoff-Vorprodukt vom Kuppler-Typ enthalten.

Es kann erfindungsgemäß bevorzugt sein, als Entwicklerkoruponente ein p-Phenylendiaminderivat oder eines seiner physiologisch verträglichen Salze einzusetzen. Besonders bevorzugt sind p-Phenylendiaminderivate der Formel (E1) 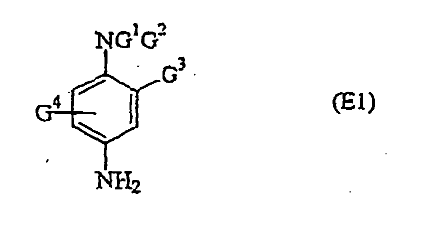 wobei
- G¹ steht für ein Wasserstoffatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen 4'-Aminophenylrest oder einen C₁-C₄-Alkylrest, der mit einer stickstoffhaltigen Gruppe, einem Phenyl- oder einem 4'-Aminophenylrest substituiert ist,
- G² steht für ein Wasserstoffatom, einen C₁-C₄-Alkyrest, einen C₁-C₄-Monohydroxyalkylrest, einen C₂-C₄-Polyhydroxyalkylrest, einen (C₁-C₄)-Alkoxy-(C₁-C₄)-alkylrest oder einen C₁-C₄-Alkylrest, der mit einer stickstoffhaltigen Gruppe substituiert ist,
- G³ steht für ein Wasserstoffatom, ein Halogenatom, wie ein Chlor-, Brom-, Iod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Acetylaminoalkoxyrest, einen C₁-C₄-Mesylaminoalkoxyrest oder einen C₁-C₄-Carbamoylaminoalkoxyrest,
- G⁴ steht für ein Wasserstoffatom, ein Halogenatom oder einen C₁-C₄-Alkykest oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Ein Beispiel für eine bevorzugte C₂-C₄-Polyhydroxyalkylgruppe ist die α,β-Dihydroxyethylgruppe. Beispiele für stickstoffhaltige Gruppen der Formel (E1) sind insbesondere die Aminogruppen, C₁-C₄-Monoalkylaminogruppen, C₁-C₄-Dialkylaminogruppen, C₁-C₄-Trialkylammoniumgruppen, C₁-C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusamnnensetzungen gemäß der Erfindung verwendet werden können, kann man insbesondere die Verbindungen nennen, die der folgenden Formel (E2) entsprechen, sowie ihre physiologisch verträglichen Salze: 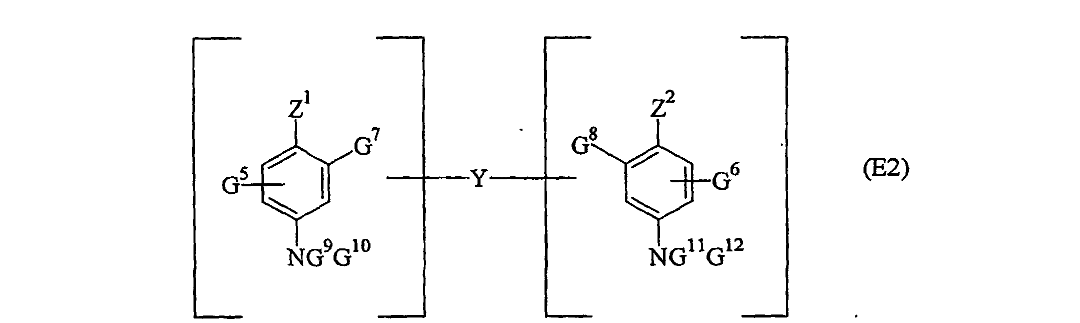 wobei:
- Z¹ und Z² stehen unabhängig voneinander für einen Hydroxyl- oder NH₂-Rest, das gegebenenfalls durch einen C₁-C₄-Alkylrest, durch einen C₁-C₄-Monohydroxyalkylrest und/oder durch eine Verbrückung Y substituiert ist oder das gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Aminoalkyhrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest,
mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetra-methylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Bis-(ethyl)-N,N'-bis-(4'-amino-3'-methylphenyl)-ethylendiamin, Bis-(2-hydroxy-5-aminophenyl)-methan, 1,4-Bis-(4'-aminophenyl)-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 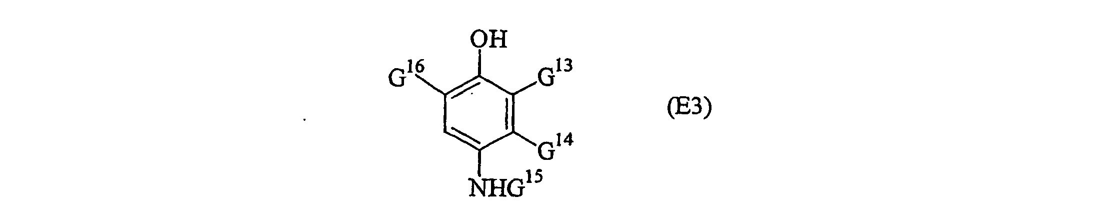 wobei
- G¹³ steht für ein Wasserstoffatom, ein Halogenatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-bis C₄)-aminoalkylrest oder einen (Di-C₁-C₄-Alkylamino)-(C₁-C₄)-alkylrest, und
- G¹⁴ steht für ein Wasserstoff- oder Halogenatom, einen C₁-C₄-Alkylrest, einen C₁-C₄-Hydroxyalkylrest, einen C₂-C₄-Polyhydroxyalkylrest, einen (C₁- C₄)-Alkoxy-(C₁-C₄)-alkylrest, einen C₁-C₄-Aminoalkylrest oder einen C₁- C₄-Cyanoalkylrest,
- G¹⁵ steht für Wasserstoff, einen C₁-C₄-Alkylrest, einen C₁-C₄-Hydroxyalkylrest, einen C₂-C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-Aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethyl-aminomethyl)phenol, 4-Amino-2-fluorphenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen. Bevorzugt werden erfindungsgemäß Pyrimidin oder Pyrazolderivate.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 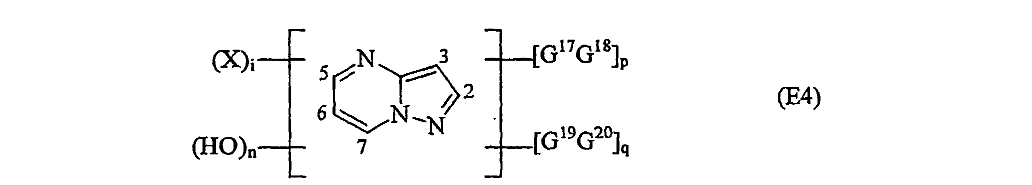 wobei:
- G¹⁷, G¹⁸, G¹⁹ und G²⁰ unabhängig voneinander stehen für ein Wasserstoffatom, einen C₁-C₄-Alkylrest, einen Aryl-Rest, einen C₁-C₄-Hydroxyalkylrest, einen C₂-C₄-Polyhydroxyalkylrest einen (C₁-C₄)-Alkoxy-(C₁-C₄)-alkylrest einen C₁-C₄-Aminoalkylrest, das gegebenenfalls durch einen Acetyl-Ureid- oder Sulfonyl-Rest geschützt sein kann, einen (C₁-C₄)-Alkylalnino-(C₁-C₄)-alkylrest, einen Di-[(C₁-C₄)-alkyl]-(C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄)-[Hydroxyalkyl]-(C₁-C₄)-aminoalkylrest,
- die X-Reste stehen unabhängig voneinander für ein Wasserstoffatom, einen C₁-C₄-Alkylrest, einen Aryl-Rest, einen C₁-C₄-Hydroxyalkylrest, einen C₂-C₄-Polyhydroxyalkylrest, einen C₁-C₄-Aminoalkylrest, einen (C₁-C₄)-Alkylamino-(C₁-C₄)-alkylrest, einen Di-[(C₁-C₄)alkyl]- (C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroayalkyl- oder einen Di-(C₁-C₄-hydroxyalkyl)-aminoalkylrest, einen Aminorest, einen C₁-C₄-Alkyl- oder Di-(C₁-C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

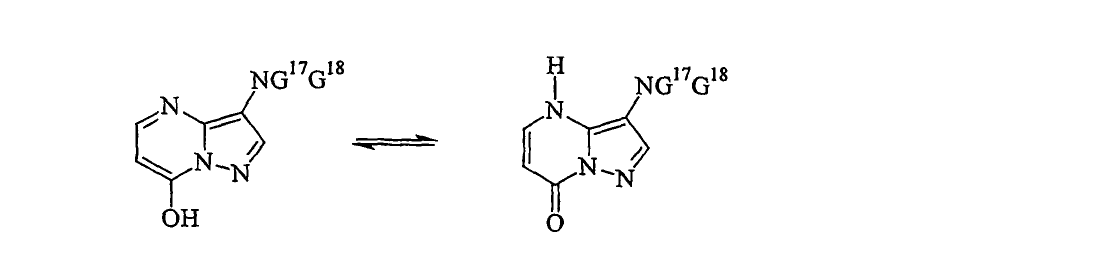

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß bevorzugte Entwicklerkomponenten sind Derivate des p-Phenylendiamins, Derivate des Pyrimidins, Derivate des Pyrazols und des p-Aminophenols.

Besonders bevorzugte Entwicklerkomponenten sind erfindungsgemäß p-Phenylendiamin, p-Toluylendiamin, 2-(β-Hydroxyethyl)-p-phenylendiamin, N,N-Bis-(β-hydroxyethyl)-p-phenylendiamin, 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 4,5-Diamino-1-methyl-pyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol, p-Aminophenol, 4-Amino-3-methylphenol, 4-Amino-3-fluorphenol, 4-Amino-2-aminomethylphenol und 4-Amino-2-((diethylamino)methyl)phenol.

Ganz besonders bevorzugte Entwicklerkomponenten sind erfindungsgemäß p-Phenylendiamin, p-Toluylendiamin, 2-(β-Hydroxyethyl)-p-phenylendiamin und N,N-Bis-(β-hydroxyethyl)-p-phenylendiamin, 2,4,5,6-Tetraaminopyrimidin, 4,5-Diamino-1-(β-hydroxyethyl)-pyrazol, 4-Amino-3-methylphenol und 4-Amino-2-aminomethylphenol

Als Kupplerkomponenten werden erfindungsgemäß insbesondere m-Phenylendiaminderivate, Naphthole, Resorcin und Resorcinderivate, Pyrazolone, und m-Aminophenole verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)propan, 1-Methoxy-2-amino-4-(2'-hydroxyethylamino)-benzol, 1,3-Bis-(2',4'-diaminophenyl)propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders bevorzugte Kupplerkomponenten sind 2-Amino-3-hydroxypyridin, 2-Amino-3-hydroxy-5-chlorpyridin, 3-Amino-2-methylamino-6-methoxy-pyridin, 3,5-Diamino-2,6-dimethoxypyridin, 2,6-Dihydroxy-3,4-dimethylpyridin, m-Phenylendiamin, 2,6-Bis-(2-hydroxyethylamino)-toluol, 3-Amino-2,4-dichlorphenol, 3-Amino-2-chlor-6-methylphenol, 5-Amino-4-chlor-2-methylphenol, 5-(β-Hydroxyethyl)-amino-2-methylphenol, 5-Amino-2-methylphenol, 2-Methylresorcin, 2-(2',4'-Diaminophenoxy)ethanol, 1,3-Bis-(2',4'-diamino-phenoxy)propan, Resorcin, 4-Chlorresorcin, Resorcinmonomethylether, m-Aminophenol, 1,7-, 2,7- und 1,5-Dihydroxynaphthalin sowie 4-Hydroxyindol und 6-Hydroxyindol.

Die erfindungsgemäßen Haarfärbemittel enthalten sowohl die Entwicklerkomponenten als auch die Kupplerkomponenten bevorzugt in einer Menge von 0,005 bis 10 Gew.% vorzugsweise von 0,1 bis 5 Gew.-% jeweils bezogen auf das gesamte Oxidationsfärbemittel.

Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer zweiten Ausführungsform wird ein Mittel zum Färben von Keratinfasern, insbesondere dem menschlichen Haar, beansprucht, enthaltend mindestensein Oxidationsmittel und / oder ein Enzven sowie mindestens ein Indol- und/oder Indolinderivat, dadurch gekennzeichnet, daß es Pyridoxin oder eines der entsprechenden physiologisch verträglichen Salze enthält.

Als Vorstufen naturanaloger Farbstoffe werden in dem erfindungsgemäßen Mittel der zweiten Ausführungsform bevorzugt Indole und/oder Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIIa), 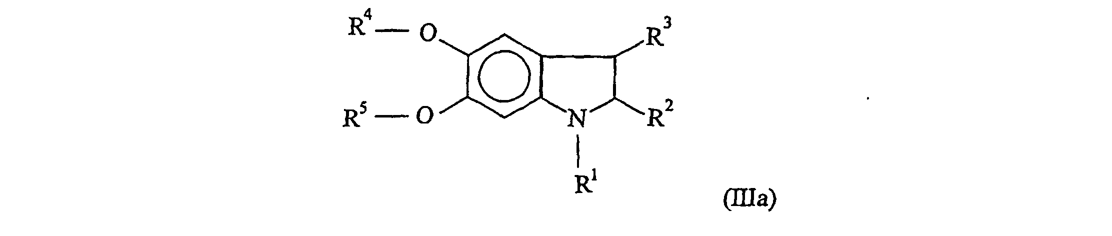 in der unabhängig voneinander
R¹ steht für Wasserstoff, eine C₁-C₄-Alkylgruppe, eine C₃-C₆-Cycloakylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIIb), 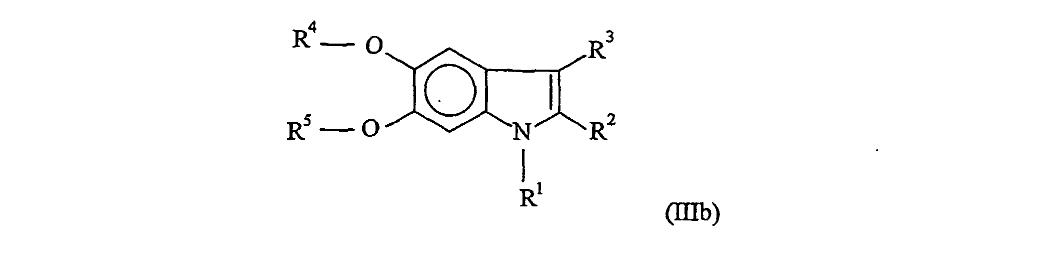 in der unabhängig voneinander
R¹ steht für Wasserstoff, eine C₁-C₄-Alkylgruppe, eine C₃-C₆-Cycloalkylgruppe, eine
C₂-C₄-Alkenylgruppe oder eine C₁-C₄-Hydroxyakylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

Fakultativ kann auch mindestens ein Oxidationsfarbstoff-Vorprodukt vom Entwickler-Typ enthalten sein. Die bevorzugten Entwickler und die davon eingesetzten Mengen entsprechen denen der ersten Ausführungsform.

In einer dritten Ausführungsform enthalten die erfindungsgemäßen Haarfärbemittel der ersten und zweiten Ausführungsform zur weiteren Modifizierung der Farbnuancen neben den Oxidationsfarbstoff-Vorprodukten zusätzlich übliche direktziehende Farbstoffe.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ebenfalls als direktziehende Farbstoffe erfindungsgemäß einsetzbar sind kationische direktziehende Farbstoffe. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 offenbart werden.

Bevorzugte kationische direktziehende Farbstoffe der Gruppe (c) sind insbesondere die folgenden Verbindungen:

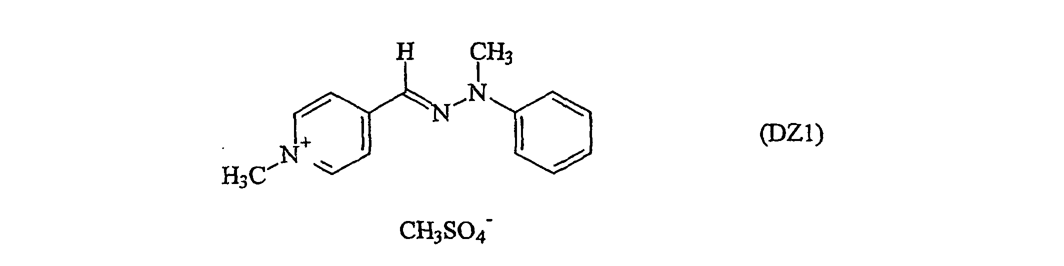

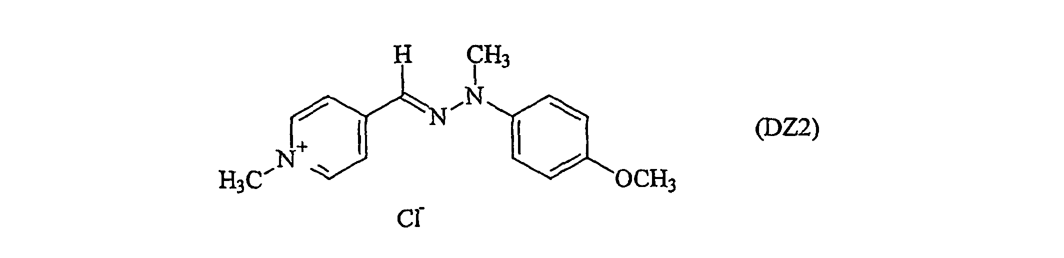

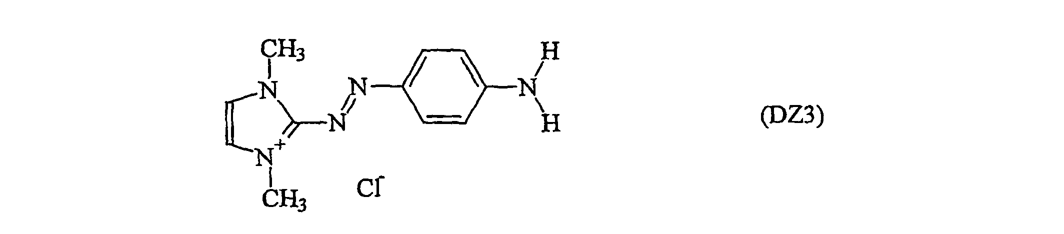

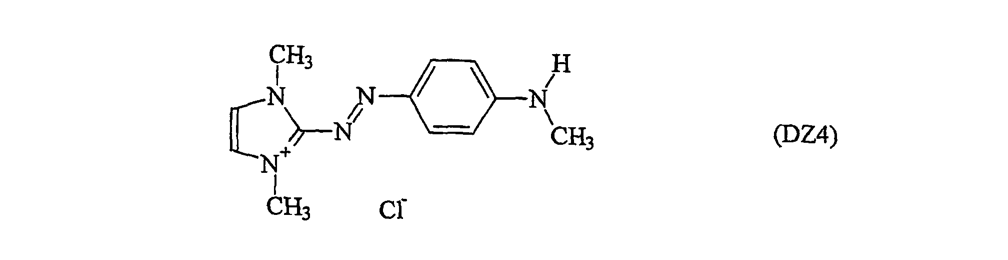

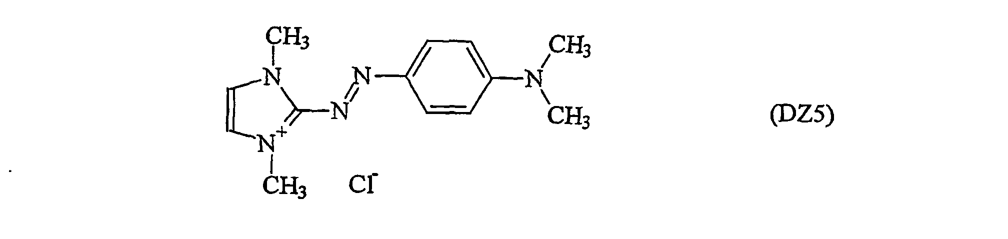

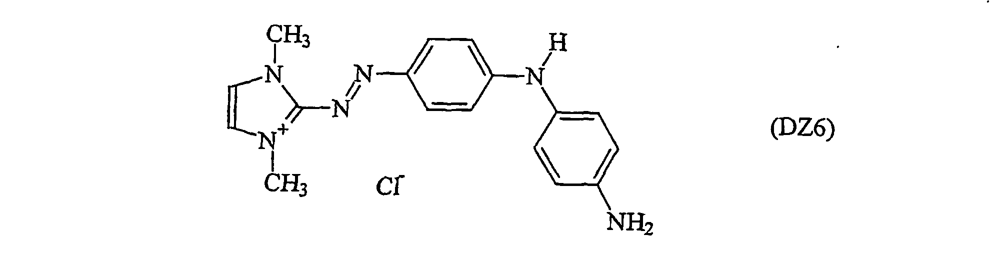

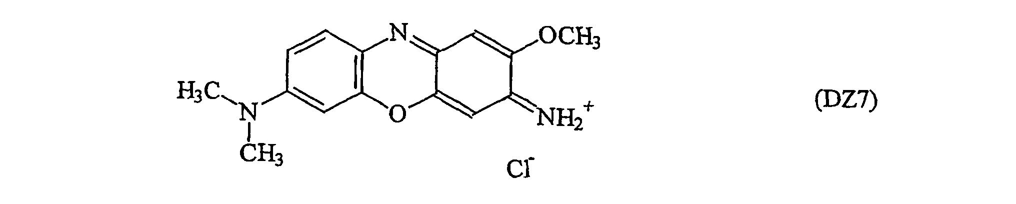

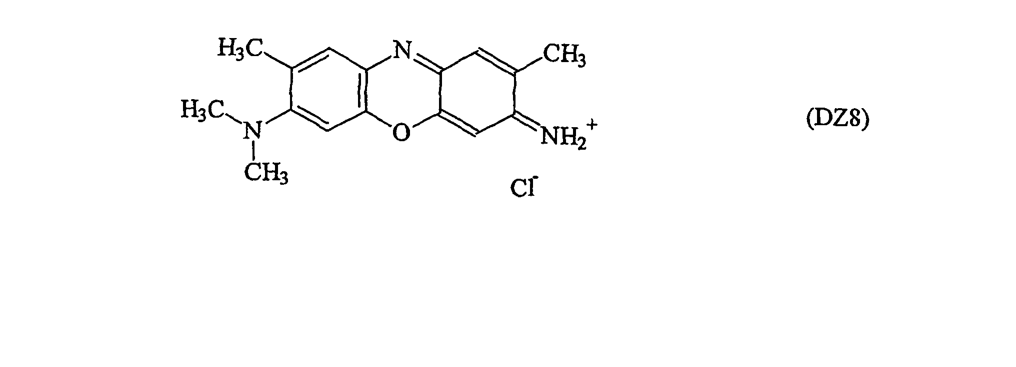

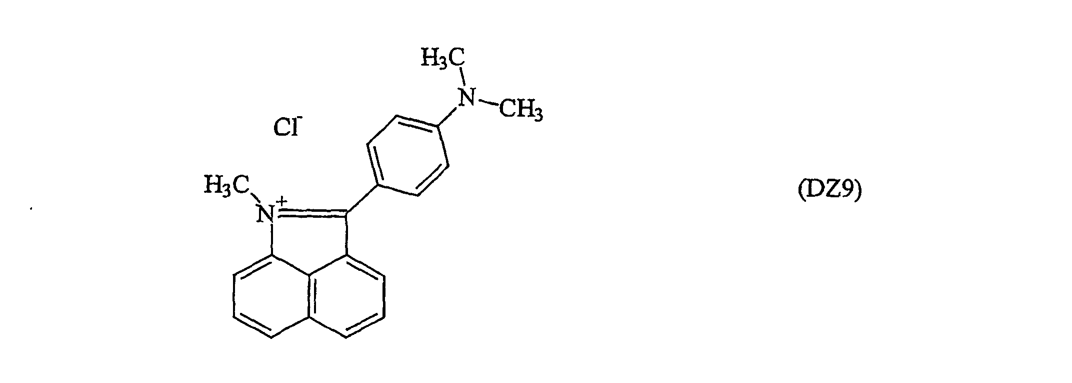

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß besonders bevorzugte direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Alle erfindungsgemäßen Färbemittel enthalten die erfindungsgemäßen Verbindungen gemäß Formel (I) bevorzugt in einer Menge von 0.05-5 Gew.%, insbesondere 0.1-1 Gew.%, bezogen auf das gesamte Mittel.

Weiterhin können die erfindungsgemäßen Zubereitungen auch in der Natur vorkommende Farbstoffe enthalten, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind.

Die erfindungsgemäßen Mittel enthalten Farbstoffvorprodukte bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch tablettenförmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase, Ascorbat-Oxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel RO-(Z)ₓ. Diese Verbindungen sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Akylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine
-COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethyl-ammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxcylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Laviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquatermium-10 und Polyquaternium-22.

Alternativ zu den kationischen Polymeren werden als konditionierende Wirkstoffe zwitterionische oder ampholytische Polymere besonders bevorzugt eingesetzt. Bevorzugte Vertreter sind Octylacrylamid/Methylmethacrylat/tert-Butylaminoethylmethacrylat/2-Hydroxypropylmethacrylat Copolymere und insbesondere das Acrylamidopropyl-trimethylammoniumchlorid/Acrylat Copolymer.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie den eingesetzten Mengen dieser Komponenten wird ausdrücklich auf die dem Fachmann bekannten einschlägigen Handbücher, z. B. K. Schrader, Grundlagen und Rezepturen der Kosmetika, 2. Auflage, Hüthig Buch Verlag, Heidelberg, 1989, verwiesen.

Ein dritter Gegenstand der Erfindung ist ein Kit zur Verwendung in einem Verfahren zum Färben von Keratinfasern, insbesondere dem menschlichen Haar. Die erste Ausführungsform ist dadurch gekennzeichnet, daß das Kit aus folgenden getrennt abgepackten Komponenten besteht:
- ein Vorbehandlungsmittel M1, enthaltend Pyridoxin oder eines des entsprechenden physiologish verträglichen Salze davon
- eine Färbemittelkomponente M2a, enthaltend mindestens ein Oxidationsfarbstoff-Vorprodukt vom Entwickler-Typ und/oder ein Indol- und/oder Indolinderivat, sowie gegebenenfalls mindestens ein Oxidationsfarbstoff-Vorprodukt vom Kuppler-Typ, und
- ein Mittel M2b, enthaltend ein Oxidationsmittel und/oder Enzym.

In einer zweiten Ausführungsform wird ein Kit beansprucht, bestehend aus folgenden getrennt abgepackten Komponenten:
- eine Färbemittelkomponente M2a, enthaltend mindestens ein Oxidationsfarbstoff-Vorprodukt vom Entwickler-Typ und/oder ein Indol- oder Indolinderivat sowie gegebenenfalls mindestens ein Oxidationsfarbstoff-Vorprodukt vom Kuppler-Typ und/oder einen direktziehenden Farbstoff,
- ein Mittel Mb2, enthaltend ein Oxidationsmittel und/oder ein Enzym,
- ein Mittel M3, enthaltend Pyridoxin oder eines der entsprechenden physiologisch verträglichen Salze.

Wird in dem Mittel M2b ein Enzym gegebenenfalls zusammen mit einem Oxidationsmittel verwendet, so ist es erfindungsgemäß bevorzugt, M2b in festem Zustand als Pulver zu konfektionieren.

Ein vierter Gegenstand der Erfindung ist die Verwendung der erfindungsgemäßen Kits in einem Verfahren zum Färben von Keratinfasern, insbesondere dem menschlichen Haar.

Die folgenden Beispiele sollen den Gegenstand der vorliegenden Anmeldung verdeutlichen.

### Beispiele

### 1. Färbebeipiele

### A) Vergleichsausfärbungen

### A1) Herstellung der Färbecremes

Zur Herstellung der Referenz-Färbecreme bzw. der Vergleichs-Färbecreme wurde stets eine Emulsion (Teilmischung A) und eine Teilmischung B präpariert. Die Referenz-Färbecreme enthält lediglich eine einzige Entwicklerkomponente. Dieser Entwickler wurde zur Anfertigung der Vergleichs-Färbecreme mit einer Verbindung gemäß Formel (I) im Stoffmengenverhältnis 1:1 kombiniert (Tabelle 1, Bsp. 1.A.1 - 1.A.5). In einem Kontrollexperiment wurde nur die Verbindung gemäß Formel (I) eingesetzt (Tabelle 1, Bsp. 1.A.6).

### Teilmischung A:

| | |
|---|---|
| Hydrenol®D¹ | 8,50 g |
| Kokoslorol®C12-18² | 2,00 g |
| Eumulgin®B2³ | 0,75 g |
| Texapon®NSO⁴ | 20,00 g |
| Dehyton®K⁵ | 12,50 g |
| Wasser | 30,00 g |

| | |
|---|---|
| ¹ C₁₆-C₁₈Fettalkohol (INCI-Bezeichnung: Cetearyl alcohol) (COGNIS) | |
| ² C₁₂-C₁₈-Fettalkohol (INCI-Bezeichnung: Coconut alcohol) (COGNIS) | |
| ³ Cetearylstearylalkohol mit ca. 20 EO-Einheiten (INCI-Bezeichnung: Ceteareth-20) (COGNIS) | |
| ⁴ Laurylethersulfat, Natriumsalz (ca. 27,5 % Aktivsubstanz; (INCI-Bezeichnung: Sodium Laureth Sulfate) (COGNIS) | |
| ⁵ N,N-Dimethyl-N-(C₈-C₁₈-kokosamidopropyl)ammoniumacetobetain (ca. 30 % Aktiv-substanz; INCI-Bezeichnung: Aqua (Water), Cocamidopropyl Betaine) (COGNIS) | |

Die Substanzen Hydrenol®D, Kokoslorol®C12-18, Eumulgin®B2, Texapon®NSO und Dehyton®K wurden bei 80°C aufgeschmolzen, mit dem 80°C heißem Wasser vermischt und unter starkem Rühren emulgiert. Danach wurde die Emulsion unter schwachem Rühren abgekühlt.

### Teilmischung B

| | |
|---|---|
| Natriumsulfit | 1,00 g |
| Ammoniumsulfat | 1,00 g |
| Farbstoffvorprodukte | wie in Tabelle 1 angegeben |
| Ammoniak (25 %-ige wäßrige Lösung) | ad pH 10 |
| Wasser | 10,00 g |

Die Farbstoffvorprodukte wurden in dem 50°C heißen Wasser unter Zugabe von Natriumsulfit und Ammoniumsulfat gelöst. Der pH-Wert wurde mit Ammoniak auf pH 10 eingestellt.

Die Teilmischung B wurde unter Rühren zu der Teilmischung A gegeben und die erhaltene Färbecreme mit Wasser auf 100g aufgefüllt und auf Raumtemperatur abgekühlt.

### A2) Färbung der Fasern

50 ml der nach A1) erhaltenen Färbecreme wurde mit jeweils 100 ml einer 3%igen H₂O₂-Lösung vermischt und auf je 5 cm lange Strähnen von "Euronaturhaar blond" (Kerling) aufgetragen. Nach 30 Minuten Einwirkungszeit bei 32°C wurde das Haar gespült, mit einem üblichen Haarwaschmittel ausgewaschen und anschließend getrocknet.
Die Ergebnisse sind der Tabelle 1 zu entnehmen. Die aufgeführten CIELAB-Koordinaten sind ein Maß für L (Helligkeit), a (Farbe Rot-Grün-Anteil), b (Farbe Gelb-Blau-Anteil), C (Buntheit) und h (Buntton) und berechnen sich aus den Normfarbwerten X, Y, Z, welche sich wiederum aus den Spektralverteilungen des Reflexionsgrades der Probe ergeben (H. G. Völz, Industrielle Farbprüfung, VCH, Weinheim, 1990.).

**Tabelle 1:**

| **Beispiel** | **Entwickler / Menge [mmol]** | **Pyridoxin·HCl Menge [mmol]** | **erhaltene Nuance** | **L** | **a** | **b** | **C** | **h** |
|---|---|---|---|---|---|---|---|---|
| 1.A.1 | E1 / 7,5 | | dunkelbraun | 38 | 9 | 18 | 20 | 64 |
| 1.A.2 | E1 / 15,0 | | senfbraun | 29 | 9 | 13 | 16 | 56 |
| 1.A.3 | E1/7,5 | 7,5 | gelbbraun | 41 | 10 | 25 | 27 | 68 |
| 1.A.4 | E2 / 3,0 | | braun | 37 | 7 | 14 | 16 | 62 |
| 1.A.5 | E2 / 3,0 | 3,0 | gelbbraun | 43 | 12 | 26 | 28 | 65 |
| 1.A.6 | | 15,0 | blassgelb | 67 | 4 | 25 | 25 | 81 |
| E1 4-Amino-2-aminomethyl-phenol·2 HCl | | | | | | | | |
| E2 p-Toluylendiamin·H₂SO₄ | | | | | | | | |

### B) Färbebeispiele mit Pyridoxin in Kombination mit mehreren Kupplern und Entwicklern

### B1) Herstellung der Färbecremes

Zur Herstellung der Färbecremes 1 und 2 wurde eine Emulsion (Teilmischung A) und die Teilmischungen B für die Färbecreme 1 und C für die Färbecreme 2 präpariert.

### Teilmischung A:

| | |
|---|---|
| Hydrenol®D¹ | 8,50 g |
| Kokoslorol®C12-18² | 2,00 g |
| Eumulgin®B2³ | 0,75 g |
| Texapon®NSO⁴ | 15,00 g |
| Dehyton®K⁵ | 12,50 g |
| Wasser | 30,00 g |

| | |
|---|---|
| ¹ C₁₆-C₁₈-Fettalkohol (INCI-Bezeichnung: Cetearyl alcohol) (COGNIS) | |
| ² C₁₂-C₁₈-Fettalkohol (INCI-Bezeichnung: Coconut alcohol) (COGNIS) | |
| ³ Cetearylstearylalkohol mit ca. 20 EO-Einheiten (INCI-Bezeichnung: Ceteareth-20) (COGNIS) | |
| ⁴ Laurylethersulfat, Natriumsalz (ca. 27,5 % Aktivsubstanz; (INCI-Bezeichnung: Sodium Laureth Sulfate) (COGNIS) | |
| ⁵ N,N-Dimethyl-N-(C₈-C₁₈-kokosamidopropyl)ammoniumacetobetain (ca. 30 % Aktivsubstanz; INCI-Bezeichnung: Aqua (Water), Cocamidopropyl Betaine) (COGNIS) | |

Die Substanzen Hydrenol®D, Kokoslorol®C12-18 und Eumulgin®B2 wurden bei 80°C aufgeschmolzen, mit dem 80°C heißem Wasser, enthaltend Texapon®NSO und Dehyton®K, vermischt und unter starkem Rühren emulgiert. Danach wurde die Emulsion unter schwachem Rühren abgekühlt.

### Teilmischung B (für Färbecreme 1)

| | |
|---|---|
| Natriumsulfit | 1,00 g |
| Ammoniumdihydrogenphosphat | 1,00 g |
| Turpinal¹SL® | 0,12 g |
| Ascorbinsäure | 0,40 g |
| L-Arginin | 1,00 g |
| p-Toluylendiamin H₂SO₄ | 242,3 mg |
| 2-Methylresorcin | 50,2 mg |
| 4-Chlorresorcin | 48,6 mg |
| 2-Methylamino-3-amino-6-methoxypyridin | 3,5 mg |
| Vitamin B6 (Pyridoxin HCl) | wie in Tabelle 2 angegeben |
| Ammoniak (25%ige wäßrige Lösung) | ad pH = 8,4 |
| Wasser | 10,00 g |

| | |
|---|---|
| ¹ 1-Hydroxyethan-1,1-diphosphonsäure (INCI-Bezeichnung: Etidronic Acid, Aqua (Water)) (COGNIS) | |

Die Farbstoffvorprodukte wurden in dem 50°C heißem Wasser unter Zugabe von Natriumsulfit, Ammoniumdihydrogenphosphat, Turpinal® SL, Ascorbinsäure und L-Arginin gelöst. Der pH-Wert wurde mit Ammoniak auf 8,4 eingestellt.

### Teilmischung C (für Färbecreme 2)

| | |
|---|---|
| Natriumsulfit | 0,40 g |
| Ammoniumdihydrogenphosphat | 0,80 g |
| Turpinal¹ SL® | 0,12 g |
| Ascorbinsäure | 0,40 g |
| L-Arginin | 1,00 g |
| Rodol²9R Base® | 0,10 g |
| p-Toluylendiamin·H₂SO₄ | 528,0 mg |
| 2,4,5,6-Tetraaminopyrimidin·H₂SO₄ | 528,0 mg |
| 4-Amino-3-methylphenol | 864,0 mg |
| 4-Chlorresorcin | 144,0 mg |
| 2,7-Dihydroxynaphthalin | 242,0 mg |
| 2,6-Di(hydroxyethylamino)toluol | 66,0 mg |
| Vitamin B6 (Pyridoxin-HCl) | wie in Tabelle 2 angegeben |
| Ammoniak (25%ige wäßrige Lösung) | ad pH = 8,6 |
| Wasser | 10,00 g |
| 2n HCl (wäßrige Lösung) | 3,5 ml |

| | |
|---|---|
| ¹ 1-Hydroxyethan-1,1-diphosphonsäure (INCI-Bezeichnung: Etidronic Acid, Aqua (Water)) (COGNIS) | |
| ² 6-Chlor-4-nitro-2-aminophenol (LOWENSTEIN) | |

Die Farbstoffvorprodukte wurden in dem 50°C heißen, mit 3,5 ml einer wäßrigen 2n HCl-Lösung versetzten Wasser unter Zugabe von Natriumsulfit, Ammoniumdihydrogenphosphat, Turpinal® SL, Ascorbinsäure und L-Arginin gelöst. Der pH-Wert wurde mit Ammoniak auf 8,6 eingestellt.

Die Färbecreme 1 wurde durch Zugabe von Teilmischung B zu 50 g der Emulsion (Teilmischung A) und anschließendem Auffüllen mit Wasser auf 100 g hergestellt. Die Präparation der Färbecreme 2 verlief analog, jedoch wurde statt Teilmischung B die Teilmischung C verwendet.

### B2) Färbung der Fasern

50 ml der nach B1) erhaltenen Färbecremes wurden mit jeweils 40 ml einer 5%igen H₂O₂-Lösung vermischt und auf je 5 cm lange Humanhaarsträhnen (Naturweiß der Fa. Kerling) aufgetragen. Nach 30 Minuten Einwirkungszeit bei 32°C wurde das Haar gespült, mit einem üblichen Haarwaschmittel ausgewaschen und anschließend getrocknet.

Die Ergebnisse sind der Tabelle 2 zu entnehmen.

**Tabelle 2:**

| **Beispiel** | **verwendete Färbecreme** | **Menge Pyridoxin·HCl** | **erhaltene Nuance** |
|---|---|---|---|
| 1.B.1 | 1 | 0,00 g | mittelaschblond |
| 1.B.2 | 1 | 0,20 g | mittelbeigeblond |
| 1.B.3 | 1 | 0,50 g | mittelbeigeblond |
| 1.B.4 | 1 | 1,00 g | mittelgoldblond |
| 1.B.5 | 2 | 0,00 g | braunrot |
| 1.B.6 | 2 | 1,00 g | braunkupferrot |
| **2. Nachweis der strukturgebenden Wirkung von Vitamin B6 bei gleichzeitiger Applikation von Vitamin B6 mit dem Färbemittel** | | | |

### A) Verwendete Analysemethode: HP-DSC (High Pressure Differential Scanning Calorimetry)

Thermoanalytische Untersuchungen eignen sich besonders zur Charakterisierung von Zweiphasensystemen, zu denen die Humanhaare als Faserkeratine mit ihrem kristallinen α-Helix-Anteil und amorphen Matrix-Anteil ebenfalls gehören. Auf der einen Seite können Glasübergänge und Alterungsverhalten der amorphen Matrix untersucht werden, auf der anderen Seite liefert das Schmelzverhalten der kristallinen, helicalen Phase wichtige Erkenntnisse. Thermoanalytische Untersuchungen sind erstmals 1899 beschrieben, erste Differenzthermoanalysen (DTA) an Proteinfasem wurden Ende der fünfziger Jahre durchgeführt (F. Schwenker, J.H. Dusenbury, Text. Res. J. 1963, 30, Seite 800 ff; W. D. Felix, M.A. McDowall, H. Eyring, ibid. (1963), 33, Seite 465 ff.). In den folgenden Jahren sind unterschiedliche thermoanalytische Meßverfahren, wie DTA, HP-DTA (High Pressure, Hochdruck-DTA) und DSC (Differential Scanning Calorimetry, Dynamische Differenz-Kalorimetrie), an Keratinfasern angewendet worden um z.B. das Phänomen der Superkontraktion, α-β-Phasenübergänge der Helices oder Denaturierungsvorgänge zu untersuchen. In jüngster Zeit wird, insbesondere am Deutschen Wollforschungsinstitut in Aachen (F.J. Wortmann, H. Deutz, J. Appl. Polym. Sci. 1993, 48, Seite 137ff.), die Methode der HP-DSC zur Untersuchung von Keratinfasern genutzt, die die Probleme mit pyrolytischen Effekten, wie sie bei der konventionellen DSC auftreten, und Probleme mit der Datenerfassung und -interpretation, wie sie die DTA birgt, ausschließt. Dabei werden DSC-Messungen an Keratinen durchgeführt, die mit Wasser in kommerziell erhältlichen, druckfesten Meßkapseln eingeschlossen sind. Im Keratin-Wasser-System entwickelt sich beim Erhitzen oberhalb von 100°C in den verkapselten Stahltiegeln ein Wasserdampfhochdruck, woraus sich die HP-DSC Analyse ableitet. Der gravierende Unterschied der HP-DSC-Thermogramme von Humanhaaren im Vergleich zu normalen DSC-Thermogrammen ist der, daß die endothermen Peaks, die den Umwandlungspunkt und Umwandlungsenthalpie wiedergeben, hier um ca. 90 °C zu niedrigeren Temperaturen verschoben sind. Das rührt daher, daß das Wasser nach Diffusion in die Haarfaser durch Schwächung und Spaltung von Wasserstoffbrücken- und Salzbindungen die Proteinstabilität vermindert und so die ,,Verleimungstemperatur" der Keratine herabsetzt. Werden durch das superkontrahierende Agens wie Wasser nur Wasserstoffbrücken und Salzbrücken gelöst, so ist der thermische Effekt reversibel (Superkontraktion). Der Vorgang wird jedoch irreversibel, sobald auch kovalente Bindungen, wie z. B. Disulfidbrücken, gespalten werden. Dies tritt ein, wenn man Humanhaarfasern in druckfesten Kapseln mit Wasser auf über 150 °C erhitzt. Die irreversible Umwandlung, interpretiert als Übergang der α-helicalen Bereiche in den Proteinen in einen ungeordneten Zustand, resultiert in endothermen Peaks, wobei die Peaklage den Umwandlungs- oder auch Denaturierungspunkt und die Peakfläche die Umwandlungs- oder Denaturierungs-Enthalpie wiedergibt

Unter Verwendung der Dynamischen Differenz-Kalorimetrie (DSC) können demnach strukturelle sowie chemische Zustände und Veränderungen in Faserkeratinen und insbesondere in Humanhaaren erfaßt werden. Unter genau definierten Versuchsbedingungen kann man bei Humanhaaren die kalorimetrisch erfaßbaren Vorgänge anhand von Thermogrammen aufzeichnen und sie bezüglich der Peaklagen, - strukturen und -flächen als Indikator für die Beeinflussung von Ordnungs-Unordnungsübergangen durch Änderungen innerer und/oder äußerer Parameter, hervorrufen z. B. durch kosmetische Behandlung der Haare, verwenden. D. h. aus den im Thermogramm von Humanhaaren aufgezeichneten endothermen Peaks lassen sich aufgrund von Peaklage (Umwandlungspunkt) und Peakfläche (Umwandlungsenthalpie) Aussagen über Festigkeit bzw. Schädigung der Humanhaarfaser treffen.

Ausführliche Untersuchungen bezüglich des Einflusses des Cystingehaltes auf die Denaturierung der α-Helices in Keratinen haben z. B. gezeigt, daß die Denaturierungs-Temperatur (Übergangstemperatur) des Keratins linear mit dem Cystingehalt ansteigt. Die erhöhte Stabilität des Matrixbereichs aufgrund des höheren Vernetzungsgrades des erhöhten Anteils an Disulfidbrücken in der Matrix führt dazu, daß die Umwandlung der in diese Matrix eingebetteten Helices erschwert wird und resultiert somit in einer Erhöhung der Denaturierungs-Temperatur. Umgekehrt kann in der Regel eine Denaturierungs-Temperatur- und vor allem Denaturierungs-Enthalpieerniedrigung bei durch Dauerwelle oder Bleichung bzw. Färbung behandelten Humanhaaren beobachtet werden (H. Deutz, Doktorarbeit, RWTH Aachen 1993).

### B) Durchführung

Humanhaar (Alkinco 6634) wurde gezielt durch eine Dauerwellbehandlung (Marktprodukt Poly Lock extra starke Dauerwelle; 40 Minuten Dauerwelle, 10 Minuten Fixieren) geschädigt. Anschließend wurde das vorbehandelte Haar mittels einer Färbecreme mit unterschiedlichem Gehalt an Pyridoxin·HCl gefärbt. Die Denaturierungstemperaturen der eingefärbten Haarproben wurden per HP-DSC thermoanalytisch bestimmt.

### B1) Herstellung des Färbegels und Färbung der Fasern

Zur Herstellung des Färbegeles wurde eine Teilmischung A und eine Teilmischung B präpariert.

### Teilmischung A:

| | |
|---|---|
| Kokoslorol®C12-18¹ | 8,00 g |
| Edenor®PK 1805² | 6,75 g |
| Texapon®NSO-UP³ | 3,50 g |
| Dehydol® LS 2⁴ | 10.10 g |
| Propylenglykol®-1,2-US | 6,75 g |
| Isopropanol | 16,50 g |

| | |
|---|---|
| ¹ C₁₂-C₁₈-Fettalkohol (INCI-Bezeichnung: Coconut alcohol) (COGNIS) | |
| ² Ölsäure (INCI-Bezeichnung: Oleic Acid) (COGNIS) | |
| ³ Laurylethersulfat, Natriumsalz (ca. 27,5 % Aktivsubstanz, pH 10-11,5; (INCI-Bezeichnung: Sodium Laureth Sulfate) (COGNIS) | |
| ⁴ C12-14-Fettalkohol+2-EO (INCI-Bezeichnung: Laureth-2) (COGNIS) ⁵ Propylenglykol (99,7%, INCI-Bezeichnung: Propylene Glycol) (REININGHAUS CHEMIE) | |

Die Komponenten der Teilmischung A wurden bei Raumtemperatur miteinander vermischt.

### Teilmischung B:

| | |
|---|---|
| Natriumsulfit | 0,40 g |
| Ascorbinsäure | 0,40 g |
| Monoethanolamin | 4,50 g |
| L-Arginin | 1,00 g |
| Gluadin® W 40¹ | 1,00 g |
| Turpinal² SL® | 0,20 g |
| p-Toluylendiamin H₂SO₄ | 0,19 g |
| 2,4,5,6-Tetraaminopyrimidin H₂SO₄ | 1,62 g |
| 4-Amino-3-Methylphenol | 57,0 mg |
| 2-Methylresorcin | 1,00 g |
| 1-(β-Hydroxyethylamino)-4-methyl-2-nitrobenzol | 0,10 g |
| Vitamin B6 (Pyridoxin HCl) | entsprechende Menge zur Erzielung des in Tabelle 3 aufgeführten Gehaltes in der Anwendungsmischung. |
| Wasser | 37,00 g |

| | |
|---|---|
| ¹ Weizenproteinhydrolysat (INCI-Bezeichnung: Aqua (Water), Hydrolized Wheat Protein, Sodium Benzoate, Phenoxyethanol, Methylparaben, Propylparaben)(COGNIS) | |
| ² 1-Hydroxyethan-1,1-diphosphonsäure (INCI-Bezeichnung: Etidronic Acid, Aqua (Water)) (COGNIS) ³ 6-Chlor-4-nitro-2-aminophenol·HCl (LOWENSTEIN DYES) | |

Das Färbegel wurde durch Zugabe der Teilmischung B zu Teilmischung A hergestellt.

### B2) Färbung und Analyse der Haarproben

50 ml eines nach B1) präparierten Färbegels wurden mit 40 ml einer 5%igen H₂O₂-Lösung vermischt (pH-Wert der Mischung: 9.5) und auf 0,5 g geschädigtes Humanhaar aufgetragen.

Nach 30 Minuten Einwirkungszeit bei 32°C wurde das Haar gespült und anschließend getrocknet. Mittels HP-DSC-Messungen wurde dann auf restrukturierende Effekte der Wirkstoffe geprüft. Die erhaltenen Denaturierungstemperaturen sind in Tabelle 3 aufgelistet.

**Tabelle 3**

| **Beispiel** | **Menge Pyridoxin HCl** | **Denaturierungstemperatur [°C]** |
|---|---|---|
| 2.1 | 0,00 g | 141,6 |
| 2.2 | 0,20 g | 141,7 |
| 2.3 | 0,50 g | 142,4 |
| 2.4 | 1,00 g | 143,0 |

Die Denaturierungstemperatur der ungefärbten, geschädigten Referenzhaarprobe betrug 147,2°C.

## Patentansprüche

1. Verfahren zum Färben von Keratinfasern, insbesondere dem menschlichen Haar, bei dem
- gewünschtenfalls ein Vorbehandlungsmittel M1 auf die Faser aufgebracht wird, dann
- ein Färbemittel M2 ,enthaltend mindestens ein Indol- und/oder Indolinderivat und/oder ein Oxidationsfarbstoff-Vorprodukt vom Entwickler - Typ sowie ein Oxidationsmittel und/oder ein Enzven, auf der Faser zur Anwendung kommt, das gegebenenfalls unmittelbar vor dem Auftragen auf die Faser aus:
Komponente M2a, enthaltend mindestens ein Indol- und/oder Indolinderivat und/oder ein Oxidationsfarbstoff-Vorprodukt vom Entwickler-Typ und
Komponente M2b, enthaltend ein Oxidationsmittel und/oder ein Enzym
gemischt wird, wobei gewünschtenfalls den einzelnen Mitteln M2a oder M2b vor der Mischung oder der Mischung M2 ein weiteres Mittel M3 zugegeben wird und dieses Färbemittel M2 nach einer Zeit von 5-30 Minuten von der Faser abgespült wird,
**dadurch gekennzeichnet, daß** mindestens eines der Mittel M1, M2a, M2b oder M3 Pyridoxin oder eines der entsprechenden physiologisch verträglichen Salze davon enthält.

2. Mittel zum Färben von Keratinfasern, insbesondere dem menschlichen Haar, gegebenenfalls zur Verwendung als Färbemittel M2 in einem Verfahren nach Anspruch 1, enthaltend mindestens ein Oxidationsmittel und/oder ein Enzym sowie mindestens ein Oxidationsfarbstoff-Vorprodukt vom Entwickler-Typ, **dadurch gekennzeichnet, daß** es zusätzlich Pyridoxin oder eines der entsprechenden physiologisch verträglichen Salze davon enthält.

3. Mittel nach Anspruch 2, **dadurch gekennzeichnet, daß** das Oxidationsfarbstoff-Vorprodukt vom Entwickler-Typ ausgewählt ist aus den Derivaten des p-Phenylendiamins, Derivaten des Pyrimidins, Derivaten des Pyrazols und des p-Aminophenols.

4. Mittel nach einem der Ansprüche 2 oder 3, **dadurch gekennzeichnet, daß** das Oxidationsfarbstoff-Vorprodukt vom Entwickler-Typ ausgewählt ist aus p-Phenylendiamin, p-Toluylendiamin, 2-(β-Hydroxyethyl)-p-phenylendiamin, N,N-Bis-(β-hydroxyethyl)-p-phenylendiamin, 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol, p-Aminophenol, 4-Amino-3-methylphenol, 4-Amino-3-fluorphenol, 4-Amino-2-aminomethylphenol und 4-Amino-2-((diethylamino)methyl)phenol.

5. Mittel nach einem der Ansprüche 2 bis 4, **dadurch gekennzeichnet, daß** das Oxidationsfarbstoff-Vorprodukt ausgewählt ist aus p-Phenylendiamin, p-Toluylendiamin, 2-(β-Hydroxyethyl)-p-phenylendiamin und N,N-Bis-(β-hydroxyethyl)-p-phenylendiamin, 2,4,5,6-Tetraaminopyrimidin, 4,5-Diamino-1-(β-hydroxyethyl)-pyrazol, 4-Amino-3-methylphenol und 4-Amino-2-aminomethylphenol.

6. Mittel zum Färben von Keratinfasern, insbesondere dem menschlichen Haar, gegebenenfalls zur Verwendung als Färbemittel M2 in einem Verfahren nach Anspruch 1, enthaltend mindestens ein Oxidationsmittel und/oder ein Enzym sowie mindestens ein Indol- und/oder Indolinderivat, **dadurch gekennzeichnet, daß** es Pyridoxin oder eines der entsprechenden physiologisch verträglichen Salze davon enthält.

7. Mittel nach Anspruch 6, **dadurch gekennzeichnet, daß** das Indolderivat ausgewählt ist aus 5,6-Dihydroxyindol, N-Methyl-5,6-dihydroxyindol, N-Ethyl-5,6-dihydroxyindol, N-Propyl-5,6-dihydroxyindol und N-Butyl-5,6-dihydroxyindol.

8. Mittel nach einem der Ansprüche 6 oder 7, **dadurch gekennzeichnet, daß** das Indolinderivat ausgewählt ist aus 5,6-Dihydroxyindolin, N-Methyl-5,6-dihydroxyindolin, N-Ethyl-5,6-dihydroxyindolin, N-Propyl-5,6-dihydroxyindolin und N-Butyl-5,6-dihydroxyindolin.

9. Mittel nach einem der Ansprüche 2 bis 8, **dadurch gekennzeichnet, daß** es weiterhin einen direktziehenden Farbstoff enthält.

10. Mittel nach einem der Ansprüche 2 bis 9, **dadurch gekennzeichnet, daß** es ein Acrylamidopropyl-trimethylammoniumchlorid/Acrylat Copolymer enthält.

11. Kit zur Verwendung in einem Verfahren zum Färben von Keratinfasern, insbesondere dem menschlichen Haar, **dadurch gekennzeichnet, daß** es als getrennt abgepackte Komponenten
- ein Vorbehandlungsmittel M1, enthaltend Pyridoxin oder eines der entsprechenden physiologisch verträglichen Salze davon,
- eine Färbemittelkomponente M2a, enthaltend mindestens ein Oxidationsfarbstoff-Vorprodukt vom Entwickler-Typ und/oder ein Indol- und/oder Indolinderivat, sowie gegebenenfalls mindestens ein Oxidationsfarbstoff-Vorprodukt vom Kuppler-Typ, und
- ein Mittel M2b, enthaltend ein Oxidationsmittel und/oder Enzym enthält.

12. Kit zur Verwendung in einem Verfahren zum Färben von Keratinfasern, insbesondere dem menschlichen Haar, **dadurch gekennzeichnet, daß** es als getrennt abgepackte Komponenten
- eine Färbemittelkomponente M2a, enthaltend mindestens ein Indol- oder Indolinderivat und/oder ein Oxidationsfarbstoff-Vorprodukt vom Entwickler-Typ, sowie gegebenenfalls mindestens ein Oxidationsfarbstoff-Vorprodukt vom Kuppler-Typ und/oder einen direktziehenden Farbstoff,
- ein Mittel M2b, enthaltend ein Oxidationsmittel und/oder ein Enzym und
- ein Mittel M3, enthaltend Pyridoxin oder eines der entsprechenden physiologisch verträglichen Salze davon,
enthält.

13. Verwendung des Kits nach einem der Ansprüche 11 oder 12 in einem Verfahren zur Färbung von Keratinfasern, insbesondere dem menschlichen Haar.

## Claims

1. A process for colouring keratin fibres, more particularly human hair, in which
- if desired, a pretreatment preparation M1 is applied to the fibres,
- a colorant M2 containing at least one indole and/or indoline derivative and/or an oxidation dye precursor of the primary intermediate type as well as an oxidizing agent and/or an enzyme is then used on the fibres, optionally having been mixed immediately before application to the fibres from:
a component M2a containing at least one indole and/or indoline derivative and/or an oxidation dye precursor of the primary intermediate type and
a component M2b containing an oxidizing agent and/or an enzyme,
another preparation M3 optionally being added to the individual preparations M2a or M2b before mixing or to the mixture M2 and the colorant M2 being rinsed off the fibres after a contact time of 5 to 30 minutes,
**characterized in that** at least one of the preparations M1, M2a, M2b or M3 contains pyridoxine or one of the corresponding physiologically compatible salts.

2. A preparation for colouring keratin fibres, more particularly human hair, optionally for use as the colorant M2 in the process claimed in claim 1, containing at least one oxidizing agent and/or an enzyme and at least one oxidation dye precursor of the primary intermediate type, **characterized in that** it additionally contains pyridoxine or one of the corresponding physiologically compatible salts,

3. A preparation as claimed in claim 2, **characterized in that** the oxidation dye precursor of the primary intermediate type is selected from derivatives of p-phenylenediamine, derivatives of pyrimidine, derivatives of pyrazole and p-aminophenol.

4. A preparation as claimed in claim 2 or 3, **characterized in that** the oxidation dye precursor of the primary intermediate type is selected from p-phenylenediamine, p-toluylenediamine, 2-(β-hydroxyethyl)-p-phenylenediamine, N,N-bis-(β-hydroxyethyl)-p-phenylenediamine, 2,4,5,6-tetraaminopyrimidine, 4-hydroxy-2,5,6-triaminopyrimidine, 2-hydroxy-4,5,6-triaminopyrimidine, 2-dimethylamino-4,5,6-triaminopyrimidine, 2,4-dihydroxy-5,6-diaminopyrimidine, 2,5,6-triaminopyrimidine, 4,5-diamino-1-methylpyrazole, 4,5-diamino-1-(β-hydroxyethyl)-pyrazole, 3,4-diaminopyrazole, 4,5-diamino-1-(4'-chlorobenzyl)-pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert.butyl-1-methylpyrazole, 4,5-diamino-1-tert.butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)-pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropyl pyrazole, 4,5-diamino-3-methyl-1-isopropyl pyrazole, 4-amino-5-(2'-aminoethyl)-amino-1,3-dimethylpyrazole, 3,4,5-triaminopyrazole, 1-methyl-3,4,5-triaminopyrazole, 3,5-diamino-1-methyl-4-methylaminopyrazole and 3,5-diamino-4-(β-hydroxyethyl)-amino-1-methylpyrazole, p-aminophenol, 4-amino-3-methylphenol, 4-amino-3-fluorophenol, 4-amino-2-aminomethylphenol and 4-amino-2-((diethylamino)-methyl)-phenol.

5. A preparation as claimed in any of claims 2 to 4, **characterized in that** the oxidation dye precursor is selected from p-phenylenediamine, p-toluylenediamine, 2-(β-hydroxyethyl)-p-phenylenediamine and N,N-bis-(β-hydroxyethyl)-p-phenylenediamine, 2,4,5,6-tetraaminopyrimidine, 4,5-diamino-1-(β-hydroxyethyl)-pyrazole, 4-amino-3-methylphenol and 4-amino-2-aminomethylphenol.

6. A preparation for colouring keratin fibres, more particularly human hair, optionally for use as the colorant M2 in the process claimed in claim 1 containing at least one oxidizing agent and/or an enzyme and at least indole and/or indoline derivative, **characterized in that** it contains pyridoxine or one of the corresponding physiologically compatible salts.

7. A preparation as claimed in claim 6, **characterized in that** the indole derivative is selected from 5,6-dihydroxyindole, N-methyl-5,6-dihydroxyindole, N-ethyl-5,6-dihydroxyindole, N-propyl-5,6-dihydroxyindole and N-butyl-5,6-dihydroxyindole.

8. A process as claimed in claim 6 or 7, **characterized in that** the indoline derivative is selected from 5,6-dihydroxyindoline, N-methyl-5,6-dihydroxyindoline, N-ethyl-5,6-dihydroxyindoline, N-propyl-5,6-dihydroxyindoline and N-butyl-5,6-dihydroxyindoline.

9. A preparation as claimed in any of claims 2 to 8, **characterized in that** it additionally contains a substantive dye.

10. A preparation as claimed in any of claims 2 to 9, **characterized in that** it contains an acrylamidopropyl trimethyl ammonium chloride/acrylate copolymer.

11. A kit for use in a process for colouring keratin fibres, more particularly human hair, **characterized in that** it contains
- a pretreatment preparation M1 containing pyridoxine or one of the corresponding physiologically compatible salts,
- a colouring component M2a containing at least one oxidation dye precursor of the primary intermediate type and/or an indole and/or indoline derivative and optionally at least one oxidation dye precursor of the secondary intermediate type and
- a preparation M2b containing an oxidizing agent and/or an enzyme as separately packed components.

12. A kit for use in a process for colouring keratin fibres, more particularly human hair, **characterized in that** it contains
- a colouring component M2a containing at least one indole or indoline derivative and/or an oxidation dye precursor of the primary intermediate type and optionally at least one oxidation dye precursor of the secondary intermediate type and/or a substantive dye,
- a preparation M2b containing an oxidizing agent and/or an enzyme and
- a preparation M3 containing pyridoxine or one of the corresponding physiologically compatible salts
as separately packed components.

13. The use of the kit claimed in claim 11 or 12 in a process for colouring keratin fibres, more particularly human hair.

## Revendications

1. Procédé de coloration de fibres kératiniques, en particulier de cheveux humains, dans lequel
• on applique, si on le souhaite, sur les fibres un produit de prétraitement M1, puis
• on utilise sur les fibres un colorant M2 contenant au moins un dérivé d'indol et/ou d'indoline et/ou un précurseur de colorant d'oxydation de type développeur ainsi qu'un agent oxydant et/ou une enzyme, le colorant M2 étant formé, éventuellement immédiatement avant l'application sur les fibres, par mélange :
d'un composant M2a contenant au moins un dérivé d'indol et/ou d'indoline et/ou un précurseur de colorant d'oxydation de type développeur et
d'un composant M2b contenant un agent oxydant et/ou une enzyme,
procédé dans lequel on ajoute, si on le souhaite, à l'agent M2a ou M2b pris séparément avant d'être mélangé, ou au mélange M2, un autre agent M3 et après une durée de 5-30 minutes, on élimine des fibres ce colorant M2 par rinçage,
**caractérisé en ce qu'**au moins l'un des agents M1, M2a, M2b ou M3 contient de la pyridoxine ou un des sels physiologiquement acceptables correspondants de ce composé.

2. Agent de coloration de fibres kératiniques, en particulier de cheveux humains, utilisable éventuellement comme colorant M2 dans un procédé selon la revendication 1, contenant au moins un agent oxydant et/ou une enzyme ainsi qu'au moins un précurseur de colorant d'oxydation de type développeur, **caractérisé en ce qu'**il contient en outre de la pyridoxine ou un des sels physiologiquement acceptables correspondants de ce composé.

3. Agent selon la revendication 2, **caractérisé en ce que** le précurseur de colorant d'oxydation de type développeur est choisi parmi les dérivés de p-phénylènediamine, les dérivés de pyrimidine, les dérivés de pyrazole et de p-aminophénol.

4. Agent selon l'une quelconque des revendications 2 ou 3, **caractérisé en ce que** le précurseur de colorant d'oxydation de type développeur est choisi parmi la p-phénylènediamine, la p-toluylènediamine, la 2-(β-hydroxyéthyl)-p-phénylènediamine, la N,N-bis-(β-hydroxyéthyl)-p-phénylènediamine, la 2,4,5,6-tétraaminopyrimidine, la 4-hydroxy-2,5,6-triaminopyrimidine, la 2-hydroxy-4,5,6-triaminopyrimidine, la 2-diméthylamino-4,5,6-triamino-pyrimidine, la 2,4-dihydroxy-5,6-diaminopyrimidine, la 2,5,6-triaminopyrimidine, le 4,5-diamino-1-méthylpyrazole, le 4,5-diamino-1-(β-hydroxyéthyl)-pyrazole, le 3,4-diaminopyrazole, le 4,5-diamino-1-(4'-chlorobenzyl)-pyrazole, le 4,5-diamino-1,3-diméthylpyrazole, le 4,5-diamino-3-méthyl-1-phénylpyrazole, le 4,5-diamino-1-méthyl-3-phénylpyrazole, le 4-amino-1,3-diméthyl-5-hydrazinopyrazole, le 1-benzyl-4,5-diamino-3-méthylpyrazole, le 4,5-diamino-3-tert.-butyl-1-méthylpyrazole, le 4,5-diamino-1-tert.-butyl-3-méthylpyrazole, le 4,5-diamino-1-(β-hydroxyéthyl)-3-méthylpyrazole, le 4,5-diamino-1-éthyl-3-méthylpyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthylpyrazole, le 4,5-diamino-3-hydroxyméthyl-1-isopropylpyrazole, le 4,5-diamino-3-méthyl-1-isopropyl-pyrazole, le 4-amino-5-(2'-aminoéthyl)amino-1,3-diméthylpyrazole, le 3,4,5-triaminopyrazole, le 1-méthyl-3,4,5-triaminopyrazole, le 3,5-diamino-1-méthyl-4-méthylaminopyrazole et le 3,5-diamino-4(β-hydroxyéthyl)amino-1-méthylpyrazole, le p-aminophénol, le 4-amino-3-méthylphénol, le 4-amino-3-fluorophénol, le 4-amino-2-aminométhylphénol et le 4-amino-2-((diéthylamino)méthyl)phénol.

5. Agent selon l'une quelconque des revendications 2 à 4, **caractérisé en ce que** le précurseur de colorant d'oxydation est choisi parmi la p-phénylènediamine, la p-toluylènediamine, la 2-(β-hydroxyéthyl)-p-phénylènediamine et la N,N-bis-(β-hydroxyéthyl)-p-phénylènediamine, la 2,4,5,6-tétra-aminopyrimidine, le 4,5-diamino-1-(β-hydroxyéthyl)-pyrazole, le 4-amino-3-méthylphénol et le 4-amino-2-aminométhylphénol.

6. Agent de coloration de fibres kératiniques, en particulier de cheveux humains, éventuellement utilisable comme colorant M2 dans un procédé selon la revendication 1, contenant au moins un agent oxydant et/ou une enzyme ainsi qu'au moins un dérivé d'indol et/ou d'indoline, **caractérisé en ce qu'**il contient de la pyridoxine ou un des sels physiologiquement acceptables correspondants de ce composé.

7. Agent selon la revendication 6, **caractérisé en ce que** le dérivé d'indoline est choisi parmi le 5,6-dihydroxyindol, le N-méthyl-5,6-dihydroxyindol, le N-éthyl-5,6-dihydroxyindol, le N-propyl-5,6-dihydroxyindol et le N-butyl-5,6-dihydroxyindol.

8. Agent selon la revendication 6 ou 7, **caractérisé en ce que** le dérivé d'indol est choisi parmi la 5,6-dihydroxyindoline, la N-méthyl-5,6-dihydroxyindoline, la N-éthyl-5,6-dihydroxyindoline, la N-propyl-5,6-dihydroxyindoline et la N-butyl-5,6-dihydroxyindoline.

9. Agent selon l'une quelconque des revendications 2 à 8, **caractérisé en ce qu'**il contient en outre un colorant montant directement sur la fibre.

10. Agent selon l'une quelconque des revendications 2 à 9, **caractérisé en ce qu'**il contient un copolymère chlorure d'acrylamidopropyl-triméthylammonium/acrylate.

11. Kit destiné à être utilisé dans un procédé de coloration de fibres kératiniques, en particulier de cheveux humains, **caractérisé en ce qu'**il contient, en tant que composant conditionné séparément,
- un agent de prétraitement M1, contenant de la pyridoxine ou un des sels physiologiquement acceptables correspondants de ce composé,
- un composant colorant M2a, contenant au moins un précurseur de colorant d'oxydation de type développeur et/ou un dérivé d'indol et/ou d'indoline, ainsi qu'éventuellement au moins un précurseur de colorant d'oxydation de type coupleur, et
- un agent M2b, contenant un agent oxydant et/ou une enzyme.

12. Kit destiné à l'utilisation dans un procédé de coloration de fibres kératiniques, en particulier de cheveux humains, **caractérisé en ce qu'**il contient, en tant que composant conditionné séparément,
- un composant colorant M2a, contenant au moins un dérivé d'indol ou d'indoline et/ou ou un précurseur de colorant d'oxydation de type développeur ainsi qu'éventuellement au moins un précurseur de colorant d'oxydation de type coupleur et/ou un colorant montant directement sur la fibre,
- un agent M2b, contenant un agent oxydant et/ou une enzyme, et
- un agent M3, contenant de la pyridoxine ou un des sels physiologiquement acceptables correspondants de ce composé.

13. Utilisation d'un kit selon la revendication 11 ou 12 dans un procédé de coloration de fibres kératiniques, en particulier de cheveux humains.
